# EUROPEAN PATENT APPLICATION

(11) **EP 4 732 775 A1**
(43) Date of publication of application: **29.04.2026**
(21) Application number: 25211753.6
(22) Date of filing: 28.10.2025
(51) Int. Cl.: A61B 6/00, A61B 6/58, A61B 6/51

(54) **X-RAY DEVICE CALIBRATION**

(30) Priority: 28.10.2024 US 202418928638
(71) Applicant: DENTSPLY SIRONA Inc., York, PA 17401 (US)
(72) Inventor: HUELSBUSCH, Markus, 64625 Bensheim (DE); MAUR, Susanne, 64625 Bensheim (DE); ERHARDT, Norbert, 64625 Bensheim (DE); GUENTHER, David, 64625 Bensheim (DE)
(74) Representative: Taor, Simon Edward William

(57) **Abstract**

Techniques and apparatuses are described that perform x-ray device calibration and imaging. In an example aspect the techniques and apparatuses include techniques and apparatuses to calibrate an x-ray device by performing a generalized calibration routine that is applicable to any rotation center of the x-ray device to measure calibration parameters of the x-ray device that describe at least mechanics of the x-ray device, imaging a volume by projecting an x-ray beam through the volume onto a detector based on a desired scanning trajectory or the calibration parameters to produce a plurality of x-ray images, generating an estimated projection geometry corresponding to the imaging using the calibration parameters and the desired scanning trajectory, and generating a reconstruction of the volume corresponding to the plurality of x-ray images using the estimated projection geometry and a motion artefact compensation.

## Description

### BACKGROUND

### Technical Field

The present disclosure pertains to x-ray device calibration and imaging, and more specifically to performing a generalized calibration routine that is applicable to any rotation center of an x-ray device for x-ray imaging.

### Description of the Related Art

In x-ray imaging, calibration may be performed to adjust and standardize an x-ray device for taking x-ray images. Calibration may be performed to maintain a quality of the images and to ensure that the device operates within the required specifications.

The calibration can include an alignment procedure to bring x-ray beams into proper alignment with a detector of the x-ray device and the object being imaged. Further, the voltage and current used for exposures may be calibrated to ensure correct exposure levels. In addition, the sensitivity of the detector can be adjusted to ensure uniform response across the surface of the detector.

### BRIEF SUMMARY

According to an embodiment of the present disclosure, a method includes performing x-ray device calibration and imaging by calibrating an x-ray device by performing a generalized calibration routine that is applicable to any rotation center of the x-ray device to measure calibration parameters of the x-ray device that describe at least mechanics of the x-ray device. The method further includes imaging a volume of a patient by projecting an x-ray beam through the volume onto a detector based on a desired scanning trajectory or based on the calibration parameters to produce a plurality of x-ray images. The method further includes generating an estimated projection geometry corresponding to the imaging using the calibration parameters and the desired scanning trajectory. The method also includes generating a reconstruction of the volume corresponding to the plurality of x-ray images using the estimated projection geometry and a motion artefact compensation.

In an embodiment, the calibration parameters include extrinsic calibration parameters that describe how components of the x-ray device move in space; and the calibration parameters further include intrinsic calibration parameters that describe relative positions of the components of the x-ray device to each other.

According to an embodiment of the present disclosure, an x-ray device includes a processor; and a memory, in communication with the processor, with one or more computer program instructions stored on the memory, the computer program instructions, when executed by the processor, cause the x-ray device to perform operations comprising: calibrating an x-ray device by performing a generalized calibration routine that is applicable to any rotation center of the x-ray device to measure calibration parameters of the x-ray device that describe at least mechanics of the x-ray device; imaging a volume by projecting an x-ray beam through the volume onto a detector based on a desired scanning trajectory or the calibration parameters to produce a plurality of x-ray images; and generating an estimated projection geometry corresponding to the imaging using the calibration parameters and the desired scanning trajectory. The x-ray device further generates a reconstruction of the volume corresponding to the plurality of x-ray images using the estimated projection geometry and a motion artefact compensation.

According to an embodiment of the present disclosure, a non-transitory computer readable storage medium stores computer-readable instruction that when executed by a processor of an x-ray device cause the x-ray device to: calibrate an x-ray device by performing a generalized calibration routine that is applicable to any rotation center of the x-ray device to measure calibration parameters of the x-ray device that describe at least mechanics of the x-ray device; image a volume by projecting an x-ray beam through the volume onto a detector based on a desired scanning trajectory or the calibration parameters to produce a plurality of x-ray images; and generate an estimated projection geometry corresponding to the imaging using the calibration parameters and the desired scanning trajectory. The x-ray device is further caused to generate a reconstruction of the volume corresponding to the plurality of x-ray images using the estimated projection geometry and a motion artefact compensation.

### BRIEF DESCRIPTION OF THE DRAWINGS

To easily identify the discussion of any particular element or act, the most significant digit or digits in a reference number refer to the figure number in which that element is first introduced.
FIG. 1 depicts a block diagram of a network of data processing systems in accordance with an illustrative embodiment.
FIG. 2 depicts a block diagram of a data processing system in accordance with an illustrative embodiment.
FIG. 3A depicts a sketch of an x-ray device in accordance with an illustrative embodiment.
FIG. 3B depicts a sketch of an x-ray device and an x-ray beam in accordance with an illustrative embodiment.
FIG. 4 depicts a sketch of an x-ray device illustrating a first volume and a first rotation center in accordance with an illustrative embodiment.
FIG. 5 depicts a sketch of an x-ray device illustrating a first volume and a first rotation center in accordance with an illustrative embodiment.
FIG. 6 depicts a routine 600 in accordance with an illustrative embodiment.

### DETAILED DESCRIPTION

### Overview

In the following detailed description, numerous specific details are set forth by way of examples to provide a thorough understanding of the relevant teachings. However, it should be apparent that the present teachings may be practiced without such details. In other instances, well-known methods, procedures, and/or components have been described at a relatively high level, without detail, to avoid unnecessarily obscuring aspects of the present teachings.

The illustrative embodiments recognize that an x-ray device may have a plurality of predefined rotation centers that may be used to capture x-ray images of an object such as a volume of a patient. Each predefined rotation center may correspond to a volume that is to be imaged, and each predefined rotation center may have a corresponding trajectory or path that may be followed by the detector and x-ray source of the x-ray device to image the volume. For each predefined rotation center, a calibration may be performed. Thus, for an x-ray device that has, for example, ten predefined rotation centers, ten corresponding calibration routines may be performed to calibrate the x-ray device.

Thus, illustrative embodiments disclose a method including performing a generalized calibration routine that is applicable to any rotation center. The generalized calibration routine includes traversing a trajectory that includes a linear translation to obtain calibration parameters that describe at least mechanics of the x-ray device. The illustrative embodiments further disclose imaging the volume and using the calibration parameters to estimate a projection geometry for use with a motion artefact compensation to generate a reconstruction of the imaged volume. The motion artefact compensation compensates for (reduces) error in estimating the projection geometry such that the generalized calibration routine can be used for all rotation centers to increase the quality of reconstructions obtained.

The illustrative embodiments are described with respect to certain types of machines. The illustrative embodiments are also described with respect to other scenes, subjects, measurements, devices, data processing systems, environments, components, and applications only as examples. Any specific manifestations of these and other similar artifacts are not intended to be limiting to the disclosure. Any suitable manifestation of these and other similar artifacts can be selected within the scope of the illustrative embodiments.

Furthermore, the illustrative embodiments may be implemented with respect to any type of data, data source, or access to a data source over a data network. Any type of data storage device may provide the data to an embodiment of the disclosure, either locally at a data processing system or over a data network, within the scope of the disclosure.

The illustrative embodiments are described using specific code, hardware, algorithms, designs, architectures, protocols, layouts, schematics, and tools only as examples and are not limiting to the illustrative embodiments. Furthermore, the illustrative embodiments are described in some instances using particular software, tools, and data processing environments only as an example for the clarity of the description. The illustrative embodiments may be used in conjunction with other comparable or similarly purposed structures, systems, applications, or architectures. For example, other comparable devices, structures, systems, applications, or architectures therefor, may be used in conjunction with such embodiment of the disclosure within the scope of the disclosure. An illustrative embodiment may be implemented in hardware, software, or a combination thereof.

The examples in this disclosure are used only for the clarity of the description and are not limiting to the illustrative embodiments. Additional data, operations, actions, tasks, activities, and manipulations will be conceivable from this disclosure and the same are contemplated within the scope of the illustrative embodiments.

Any advantages listed herein are only examples and are not intended to be limiting to the illustrative embodiments. Additional or different advantages may be realized by specific illustrative embodiments. Furthermore, a particular illustrative embodiment may have some, all, or none of the advantages listed above.

### Example Environment

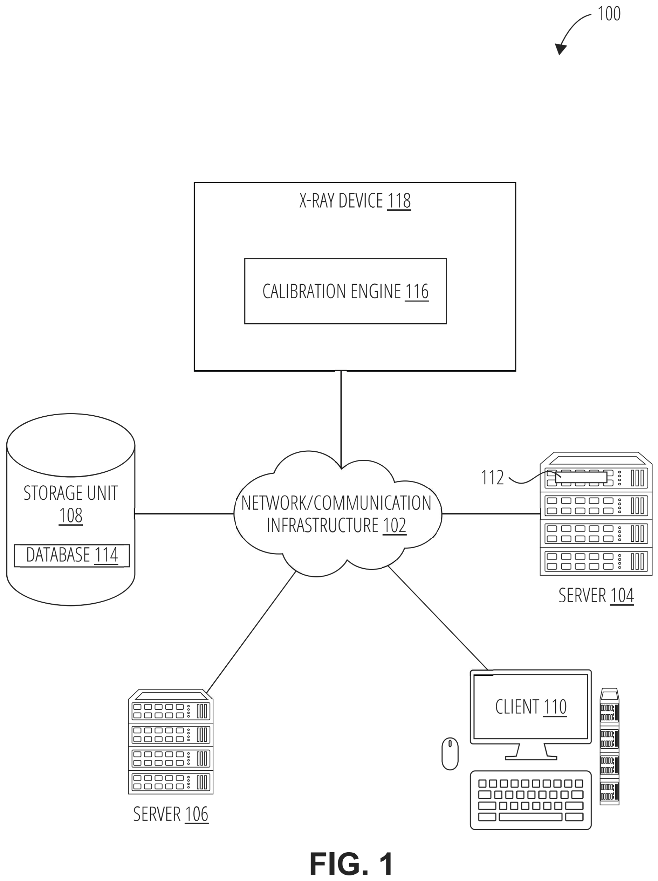

FIG. 1 depicts a block diagram of an environment of data processing systems in which illustrative embodiments may be implemented. Data processing environment 100 is a network of engines and computers in which the illustrative embodiments may be implemented. Data processing environment 100 can include network/communication infrastructure 102. Network/communication infrastructure 102 is the medium used to provide communications links between various devices, databases and computers connected together within data processing environment 100. Network/communication infrastructure 102 may include connections, such as wired connections, wireless communication protocols, or other suitable data connectivity.

The x-ray device 118 can implement embodiments described herein. The x-ray device 118 can include a calibration engine 116 which may further include an embedded application to use data of the x-ray device 118 for performing a generalized calibration routine. The embedded application can also execute in any of data processing systems (server 104 or server 106, client 110), such as client server application 112 in server 104.

X-ray radiography can be performed by positioning an x-ray source on one side of a patient (e.g., a tooth region of a patient) and causing the x-ray source to emit x-rays through the volume and toward an x-ray detector located on the other side of the patient. As the x-rays pass through the patient and volume from the x-ray source, their energies are absorbed to varying degrees depending on the composition of the volume, and x-rays arriving at the x-ray detector form a two-dimensional (2D) x-ray image or projection image (also known as a radiograph) based on the cumulative absorption through the volume.

Turning back to FIG. 1, clients or servers are only example roles of certain data processing systems connected to network/communication infrastructure 102 and are not intended to exclude other configurations or roles for these data processing systems. Server 104 and server 106 couple to network/communication infrastructure 102 along with storage unit 108. Software applications may execute on any computer in data processing environment 100. Client 110 is also coupled to network/communication infrastructure 102. A data processing system, such as server 104 or server 106, client 110 may include data and may have software applications or software tools executing thereon.

Only as an example, and without implying any limitation to such architecture, FIG. 1 depicts certain components that are usable in an example implementation of an embodiment. As another example, an embodiment can be distributed across several data processing systems and a data network as shown, whereas another embodiment can be implemented on a single data processing system within the scope of the illustrative embodiments.

Data processing environment 100 may include additional servers, clients, and other devices that are not shown. Server 104 includes the server application 112 that may be configured to implement one or more of the functions described herein for displaying restoration proposals in accordance with one or more embodiments.

In the depicted example, data processing environment 100 may be the Internet. Network/communication infrastructure 102 may represent a collection of networks and gateways that use the Transmission Control Protocol/Internet Protocol (TCP/IP) and other protocols to communicate with one another. FIG. 1 is intended as an example, and not as an architectural limitation for the different illustrative embodiments.

Among other uses, data processing environment 100 may be used for implementing a client-server environment in which the illustrative embodiments may be implemented. A client-server environment enables software applications and data to be distributed across a network such that an application functions by using the interactivity between a client data processing system and a server data processing system. Data processing environment 100 may also employ a service-oriented architecture where interoperable software components distributed across a network may be packaged together as coherent business applications. Data processing environment 100 may also take the form of a cloud, and employ a cloud computing model of service delivery for enabling convenient, on-demand network access to a shared pool of configurable computing resources (e.g. networks, network bandwidth, servers, processing, memory, storage, applications, virtual machines, and services) that can be rapidly provisioned and released with minimal management effort or interaction with a provider of the service.

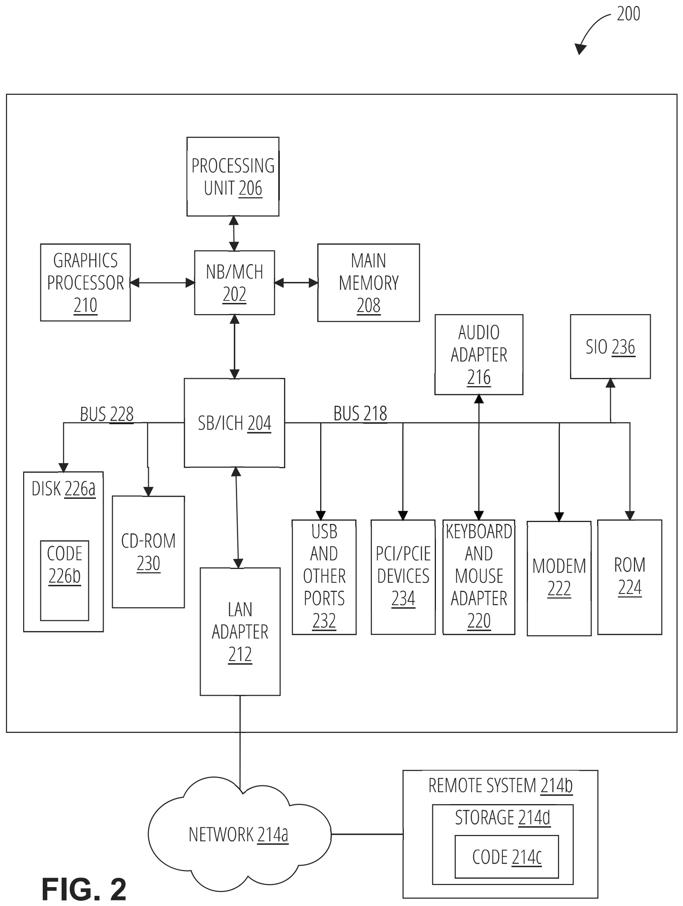

With reference to FIG. 2, this figure depicts a block diagram of a data processing system in which illustrative embodiments may be implemented. Data processing system 200 is an example of a computer, such as x-ray device 118, client 110 or server 104 in FIG. 1, or another type of device in which computer usable program code or instructions implementing the processes may be located for the illustrative embodiments.

Data processing system 200 is described as a computer only as an example, without being limited thereto. Implementations in the form of other devices, may modify data processing system 200, such as by adding a touch interface, and even eliminate certain depicted components from data processing system 200 without departing from the general description of the operations and functions of data processing system 200 described herein.

In the depicted example, data processing system 200 employs a hub architecture including North Bridge and memory controller hub (NB/MCH) 202 and South Bridge and input/output (I/O) controller hub (SB/ICH) 204. Processing unit 206, main memory 208, and graphics processor 210 are coupled to North Bridge and memory controller hub (NB/MCH) 202. Processing unit 206 may include one or more processors and may be implemented using one or more heterogeneous processor systems. Processing unit 206 may be a multi-core processor. Graphics processor 210 may be coupled to North Bridge and memory controller hub (NB/MCH) 202 through an accelerated graphics port (AGP) in certain implementations.

In the depicted example, local area network (LAN) adapter 212 is coupled to South Bridge and input/output (I/O) controller hub (SB/ICH) 204. Audio adapter 216, keyboard and mouse adapter 220, modem 222, read only memory (ROM) 224, universal serial bus (USB) and other ports 232, and PCI/PCIe devices 234 are coupled to South Bridge and input/output (I/O) controller hub (SB/ICH) 204 through bus 218. Hard disk drive (HDD) or solid-state drive (SSD) 226a and CD-ROM 230 are coupled to South Bridge and input/output (I/O) controller hub (SB/ICH) 204 through bus 228. PCI/PCIe devices 234 may include, for example, Ethernet adapters, add-in cards, and PC cards for notebook computers. PCI uses a card bus controller, while PCIe does not. Read only memory (ROM) 224 may be, for example, a flash binary input/output system (BIOS). Hard disk drive (HDD) or solid-state drive (SSD) 226a and CD-ROM 230 may use, for example, an integrated drive electronics (IDE), serial advanced technology attachment (SATA) interface, or variants such as external-SATA (eSATA) and micro- SATA (mSATA). A super I/O (SIO) device 236 may be coupled to South Bridge and input/output (I/O) controller hub (SB/ICH) 204 through bus 218.

Memories, such as main memory 208, read only memory (ROM) 224, or flash memory (not shown), are some examples of computer usable storage devices. Hard disk drive (HDD) or solid-state drive (SSD) 226a, CD-ROM 230, and other similarly usable devices are some examples of computer usable storage devices including a computer usable storage medium.

An operating system runs on processing unit 206. The operating system coordinates and provides control of various components within data processing system 200 in FIG. 2. The operating system may be a commercially available operating system for any type of computing platform, including but not limited to server systems, personal computers, and mobile devices. An object oriented or other type of programming system may operate in conjunction with the operating system and provide calls to the operating system from programs or applications executing on data processing system 200.

Instructions for the operating system, the object-oriented programming system, and applications or programs, such as server application 112 in FIG. 1, are located on storage devices, such as in the form of codes 226b on Hard disk drive (HDD) or solid-state drive (SSD) 226a, and may be loaded into at least one of one or more memories, such as main memory 208, for execution by processing unit 206. The processes of the illustrative embodiments may be performed by processing unit 206 using computer implemented instructions, which may be located in a memory, such as, for example, main memory 208, read only memory (ROM) 224, or in one or more peripheral devices.

Furthermore, in one case, code 226b may be downloaded over network 214a (such as network/communication infrastructure 102) from remote system 214b, where similar code 214c is stored on a storage device 214d in another case, code 226b may be downloaded over network 214a to remote system 214b, where downloaded code 214c is stored on a storage device 214d.

A communications unit may include one or more devices used to transmit and receive data, such as a modem or a network adapter. A memory may be, for example, main memory 208 or a cache, such as the cache found in North Bridge and memory controller hub (NB/MCH) 202. A processing unit may include one or more processors or CPUs.

Where a computer or data processing system is described as a virtual machine, a virtual device, or a virtual component, the virtual machine, virtual device, or the virtual component operates in the manner of data processing system 200 using virtualized manifestation of some or all components depicted in data processing system 200. For example, in a virtual machine, virtual device, or virtual component, processing unit 206 is manifested as a virtualized instance of all or some number of hardware processing units 206 available in a host data processing system, main memory 208 is manifested as a virtualized instance of all or some portion of main memory 208 that may be available in the host data processing system, and Hard disk drive (HDD) or solid-state drive (SSD) 226a is manifested as a virtualized instance of all or some portion of Hard disk drive (HDD) or solid-state drive (SSD) 226a that may be available in the host data processing system. The host data processing system in such cases is represented by data processing system 200.

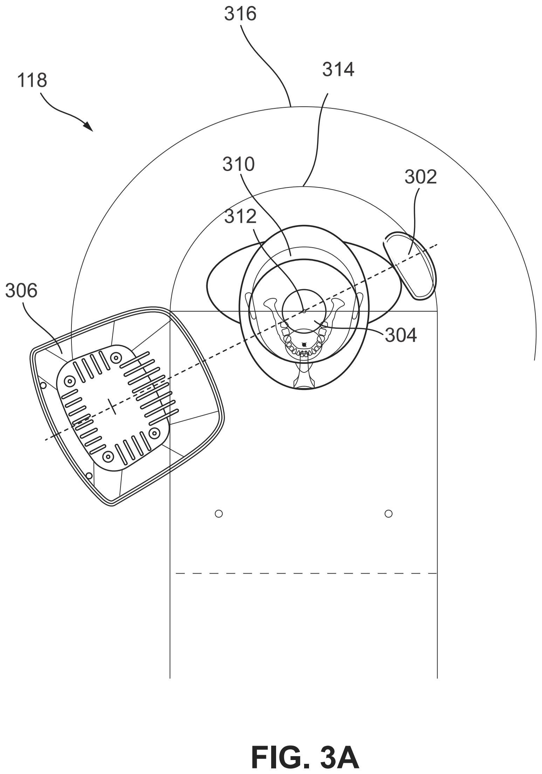

FIG. 3A shows a sketch of an operation of an x-ray device 118 in accordance with an illustrative embodiment. The x-ray device 118 comprises a detector 302, and a source 306. The x-ray device can have a plurality of rotation centers 312. A rotation center 312 is a center or reference point in a volume 304 around which the source 306 and detector 302 move for x-ray acquisition. The source and detector may move along a trajectory (source trajectory 316 and detector trajectory 314) for each rotation center 312. The rotation center 312 can be predefined. Conventionally, rotation centers may have corresponding trajectories for the source and detector through which the source and detector pass. Calibrating an x-ray device for each rotation center allows obtaining information about an actual scanning path which can deviate from a desired scanning path due to, for example, wide tolerances in how components of the x-ray device behave. Thus, the more rotation centers available, the longer the calibration process takes conventionally. The illustrative embodiments disclose a generalized calibration routine that is applicable to all rotation centers and can be performed once.

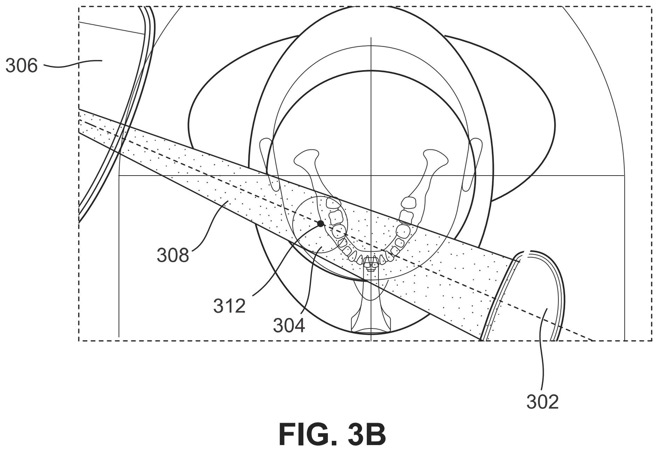

As shown in FIG. 3B, x-ray beams 308 are projected while the device rotates around a patient's head 310, in particular, around a volume 304 of the patient's head 310 or a volume 304 of another object. By performing a single generalized calibration routine as described herein, calibration parameters may be obtained for use during reconstruction such that artefacts caused by the deviations can be corrected.

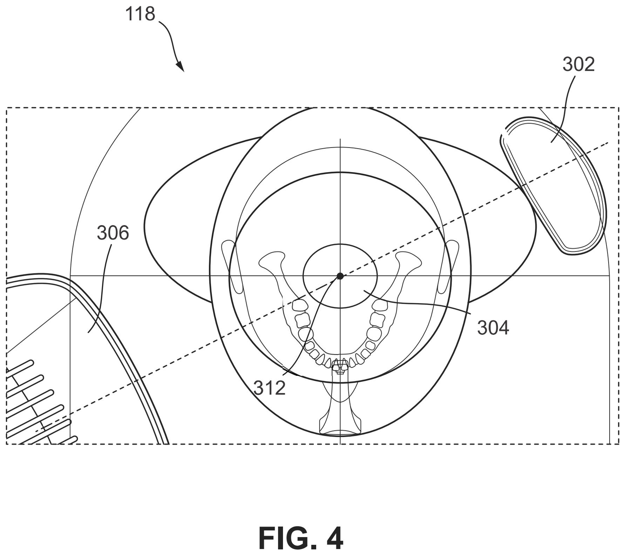

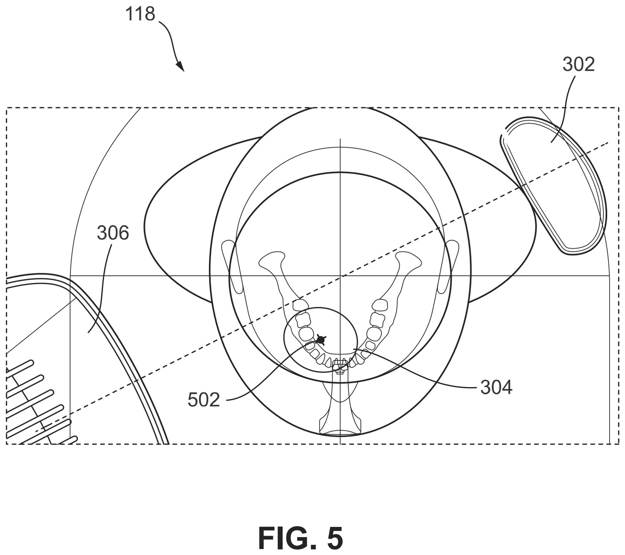

Turning to FIG. 4, the generalized calibration routine is described further. FIG. 4 shows a volume 304 with a rotation center 312. The rotation center 312 has a trajectory. Other rotation centers and trajectories such as rotation center 502 and corresponding trajectory (not shown) (*see* FIG. 5) may be available for the x-ray device 118. However, rather than using these trajectories, the generalized calibration routine can be performed. When a generalized calibration routine is performed, the trajectory used during the generalized calibration routine may also be generalized by choosing a trajectory that is not based on any particular rotation center 312. For example, the trajectory chosen for the generalized calibration routine may be at least partially non-circular. As an example, a linear translation may be performed such produce a non-circular segment of the trajectory as discussed herein. By generalizing the calibration and the corresponding trajectory, calibration parameters are generated for use in estimating projection geometries for each scan, the estimated projection geometries being usable along with a motion artefact compensation process to reduce artefacts in reconstructions. The artefacts are artefacts that may be caused by errors in the estimation of the projection geometries. By so doing, a different rotation center can be chosen at will for patient scans without necessarily relying on predetermined rotation centers.

More specifically, the illustrative embodiments disclose calibrating the x-ray device 118 by performing a generalized calibration routine for any number of rotation centers 312 of the x-ray device 118 that may or may not be predetermined rotation centers. The generalized calibration routine is performed to measure calibration parameters of the x-ray device 118 for use in subsequent estimations of projection geometries that are further utilized for volume reconstructions. The calibration parameters are parameters that describe at least mechanics of the x-ray device 118 and show how the x-ray device 118 moves. The calibration parameters may be computed for selected positions of the device trajectory during the generalized calibration routine. Generally, the calibration routine determines geometric conditions and dependencies between motor/actuator control and the projection geometry and can be used to determine projection geometries. Projection geometries may describe, for example, the position of the source 306, the position of the detector 302, how the source 306 and detector 302 rotate and move around the patient, the distance angle between the source 306 and detector, the pixel size of the detector, and other projection parameters. During the generalized calibration routine, an x-ray exposure or a series of x-ray exposures can be performed. For an x-ray exposure, x-ray beams 308 are generated at different states of the x-ray device (for example, at different motor states, motor positions, duration of use of motors, number of motors used at a same time, source/detector positions, etc.). At least one x-ray projection of the calibration routine depicts a calibration body (not shown) with known geometric properties. Further, at least one of the x-ray exposures includes configuring a motor to generate a linear movement instead of a rotational movement. This may produce the linear section of the trajectory of the generalized calibration routine. For example, by shifting the rotation center 312 from a first location to a second location during the exposure, or by performing a translational movement of the detector 302 and source 306, a non-circular segment of the trajectory taken by the detector 302 and source 306 can be obtained.

The calibration parameters include extrinsic calibration parameters that describe how components of the x-ray device move in space and/or mechanical properties of the components of the x-ray device 118. The extrinsic calibration parameters can comprise positions of the detector 302 and source 306. The extrinsic calibration parameters can also include information about how the motors are controlled to produce movements such as the linear translational movement that helps to measure device specific mechanical properties.

The calibration parameters can also include intrinsic calibration parameters that describe relative positions of the components of the x-ray device to each other. Examples of intrinsic calibration parameters include u/v shift, u0/v0 point, distance from the x-ray source to the detector, the pixel size of the detector etc. With regards to u/v shift and u0/v0 point, in general two coordinate systems may be used: a global patient-aligned 3D system with axes x, y, z with the positive z axis usually pointing towards the top of the patient, and a second coordinate system relative to the rotating detector. To avoid naming confusion, the two axes relative to the rotating detector are called u- and v-axis. The sensor v-axis usually points roughly in the same direction as the patient's z-axis. A detector shift in the imaging plane can be expressed as u/v shift. A special characteristic point describing the alignment of the detector is the u0/v0 point which marks the point, where an x-ray beam is perpendicular to the detector plane. Even further, the interaction of the extrinsic calibration parameters, the intrinsic calibration parameters and motor control properties yield parameters of the known kinematic device model. In implementations, the generalized calibration routine includes imaging the calibration body which has known geometric properties to measure the intrinsic calibration parameters. This provides the sixes and relative positions of the x-ray device components to the calibration body. For example, that calibration body can be x-ray opaque sphere-like structures that may be used in several planes, at predetermined locations per plane. In some implementations, there are at least 3 planes, and at least 4 calibration bodies per plane. Alternatively, tubes with predetermined shapes (e.g., round) and predetermined dimensions (e.g., diameter dimensions) can be used. In some implementations, the generalized calibration routine comprises no movement of the x-ray device, and the intrinsic calibration parameters are used in generating the estimated projection geometry. More specifically, if the generalized calibration routine is a single exposure (or series) without device movement, the intrinsic parameters are what can be estimated for use.

In implementations, upon completing the generalized calibration routine, volumes of patients can be imaged with any desired rotation center and the calibration parameters can be used in computing projection geometries for reconstructing the imaged patient volumes. More specifically, a volume 304 of a patient's head 310 (or other object) is imaged by projecting an x-ray beam 308 through the volume 304 onto a detector 302 based on a desired scanning trajectory or based on the calibration parameters to produce a plurality of x-ray images. For example, a practitioner may define a scan area, selects the position and size of the volume 304, and a device trajectory that can be traversed to capture the volume is determined. The device trajectory can be chosen to optimize scanning of the particular volume 304 selected by the practitioner as the volume 304 is not limited to any particular number of predetermined volumes. Optionally, the computed calibration parameters can be used to control the x-ray device 118 during imaging of the patient. This may slightly adjust motor control parameters to optimize the scanning trajectory. The calibration parameters may be applied during device control/the imaging process, during the reconstruction process, or both. Other measurement data (such as optical data from a camera showing the position of the patient) can be used to get a suitable device trajectory. In implementations, an estimate of the projection geometry may be available. For every intended movement of the device the motor control parameters are known. These could either be combined with the mechanical device construction parameters without usage of the calibration parameters or calibration parameters may be used to refine the device construction parameters.

In implementations, after scanning the patient, an estimated projection geometry corresponding to the patient scan is generated using the calibration parameters and the desired scanning trajectory. The calibration parameters help in obtaining a more accurate scanning trajectory based on the desired scanning trajectory the more accurate scanning trajectory being closer to the actual scanning trajectory. More specifically, an actual scanning trajectory of the x-ray device 118 may be different from the desired scanning trajectory. Using the calibration parameters helps to get estimates of the actual trajectory for use in reconstructing the scanned volume 304 of the patient. More specifically, the theoretical geometry of the x-ray device 118 may be known. The theoretical mechanics of the x-ray device may also be known. Thus, the theoretical movements of the x-ray device 118 can be computed. However, because of slight thresholds or offsets between different manufactured x-ray devices 118, and because of some limitations with scanning accuracy, performing the generalized calibration routine enables the generation of calibration parameters that can be used to improve the theoretical knowledge about the x-ray device 118 to more accurate actual knowledge about the x-ray device 118 without restrictions to any particular rotation center. The more accurate actual knowledge may be the estimated scanning trajectory which is used to obtain the estimated projection geometry which is also subsequently used in reconstructing the volume 304.

In implementations, upon generating the estimated projection geometry for the patient scan, a three-dimensional reconstruction of the volume 304 is generated using the estimated projection geometry and a motion artefact compensation.

More specifically, because of possible slight inaccuracies or assumptions made in the estimated projection geometry, artefacts may be present in the reconstructed volume. Thus, the reconstruction of the volume may be performed by utilizing a motion artefact compensation and the estimated projection geometry. The motion artifact correction not only corrects for motion of the patient but can also correct for errors in the assumptions about the estimated device trajectory and thus the estimated projection geometry. This is because motion artifact correction can correct for both the patient's movements and the device's movements. Due to the estimated device trajectory potentially not being 100% accurate, the motion artefact compensation can correct it also since it is a relative error (a patient moving differently than expected relative to the device is similar to the device moving differently than expected relative to the patient and both are manifested as errors in projection geometry). The motion artefact compensation is performed for individual patient scans or recordings (a plurality of x-ray projection images which are used for a volume reconstruction) and therefore helps to relax/alleviate the need for multiple calibrations of multiple rotation centers. In implementations, some classes of motion artefact compensation may be used including the following. Firstly, motion artefact compensation algorithms, which use the x-ray projection data and explicitly estimate the patient movement relative to the device. From a mathematical point of view, it is equivalent if the patient moves, or if the device movement differs from the planned trajectories. When using the estimated patient movement information, a new volume reconstruction with reduced artefacts can be performed. Secondly, motion artefact compensation algorithms which act as volume filters, but do not explicitly estimate real movement between device and patient, may be used. In the second case, transformation of an input volume with artefacts to an output volume with reduced artefacts may be performed.

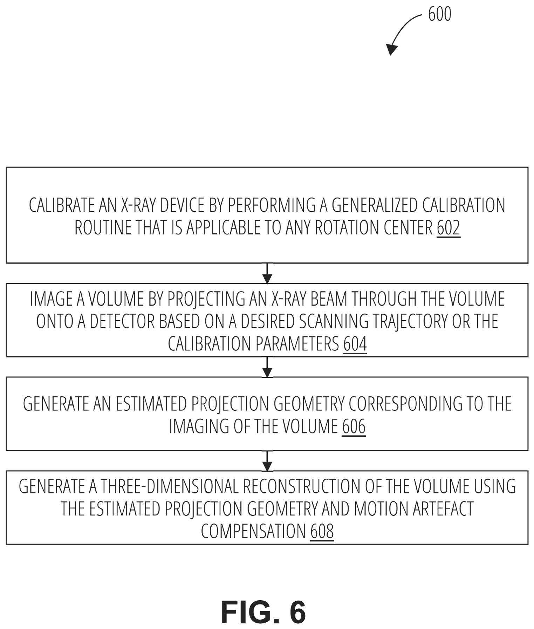

FIG. 6 illustrates a routine 600 for calibrating an x-ray device for patient measurements. The routine 600 may be performed by or in conjunction with the calibration engine 116. In block 602, the calibration engine 116 calibrates an x-ray device 118 by performing a generalized calibration routine that is application to any chosen rotation centers of the x-ray device 118. In the generalized calibration routine, calibration parameters of the x-ray device that describe at least mechanics of the x-ray device are measured. In block 604, the calibration engine 116 images a volume 304 by projecting an x-ray beam 308 through the volume 304 onto a detector 302 based on a desired scanning trajectory or based on the calibration parameters to produce a plurality of x-ray images. In block 606, the calibration engine 116 generates an estimated projection geometry corresponding to the imaging of the volume 304 using the calibration parameters and the desired scanning trajectory. This brings the estimated projection geometry closer to an actual projection geometry. In block 608, routine 600 generates a reconstruction of the volume corresponding to the plurality of x-ray images using the estimated projection geometry and a motion artefact compensation. The motion artefact compensation corrects remaining errors in the estimation of the estimated projection geometry.

Thus, a computer implemented method, system or apparatus, and computer program product are provided in the illustrative embodiments for x-ray device calibration and imaging and other related features, functions, or operations. Where an embodiment or a portion thereof is described with respect to a type of device, the computer implemented method, system or apparatus, the computer program product, or a portion thereof, are adapted or configured for use with a suitable and comparable manifestation of that type of device.

The present invention may be a system, a method, and/or a computer program product at any possible technical detail level of integration. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, including but not limited to computer-readable storage devices as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

### Conclusion

Though example systems and methods for x-ray calibration and imaging have been disclosed herein, they are not meant to be limiting as other examples can be obtained in view of the drawings and descriptions herein. For example, any combination of the following examples is possible.

Example 1: A method comprising: calibrating an x-ray device by performing a generalized calibration routine that is applicable to any rotation center of the x-ray device to measure calibration parameters of the x-ray device that describe at least mechanics of the x-ray device; imaging a volume by projecting an x-ray beam through the volume onto a detector based on a desired scanning trajectory or the calibration parameters to produce a plurality of x-ray images; generating an estimated projection geometry corresponding to the imaging using the calibration parameters and the desired scanning trajectory; and generating a reconstruction of the volume corresponding to the plurality of x-ray images using the estimated projection geometry and a motion artefact compensation.

Example 2: The method of Example 1, wherein a trajectory of the generalized calibration routine is different from the trajectory of any predefined rotation centers.

Example 3: The method of Example 1 or any other examples, wherein: the calibration parameters include extrinsic calibration parameters that describe how components of the x-ray device move in space; and the calibration parameters further include intrinsic calibration parameters that describe relative positions of the components of the x-ray device to each other.

Example 4: The method of Example 3 or any previous Examples, wherein a trajectory of the generalized calibration routine includes a non-circular segment configured to measure the extrinsic calibration parameters.

Example 5: The method of Example 3 or any previous Examples, wherein the generalized calibration routine includes imaging a calibration body with known geometric properties to measure the intrinsic calibration parameters.

Example 6: The method of Example 5 or any previous Examples, wherein: the generalized calibration routine comprises no movement of the x-ray device, and the intrinsic calibration parameters are used in generating the estimated projection geometry.

Example 7: The method of Example 1, wherein the reconstruction includes at least one of a CBCT (Cone beam computed tomography) volume, a panoramic x-ray image and a cephalometric image.

Example 8: The method of Example 1 or any other Examples, wherein the motion artefact compensation corrects artefacts in the reconstruction caused by errors in estimating the estimated projection geometry.

Example 9: The method of Example 1 or any other Examples, further comprising:
using position encoders or light barriers to obtain additional position information about components of the x-ray device and generating the estimated projection geometry based on the additional position information.

Example 10: The method of Example 1 or any other Examples, wherein the desired scanning trajectory takes into account an image quality consideration for achieving a predetermined image quality.

Example 11: The method of Example 10 any previous Examples, wherein the image quality consideration is a speed consideration or a sensor timing consideration.

Example 12: An x-ray device comprising: a processor; and a memory, in communication with the processor, with one or more computer program instructions stored on the memory, the computer program instructions, when executed by the processor, cause the x-ray device to perform any of the methods of Examples 1 - 10.

Example 13: The x-ray device of Example 12; further comprising: position encoders or light barriers configured to obtain additional position information about components of the x-ray device, and wherein the computer program instructions, when executed by the processor, cause the x-ray device to perform operations comprising generating the estimated projection geometry based on the additional position information.

Example 14: A non-transitory computer readable storage medium storing computer-readable instruction that when executed by a processor of an x-ray device cause the x-ray device to perform any of the methods of Examples 1 - 10.

Example 15: The non-transitory computer readable storage medium of Example 14, wherein the reconstruction includes at least one of a CBCT (Cone beam computed tomography) volume, a panoramic x-ray image and a cephalometric image.

## Claims

1. A method comprising:
calibrating an x-ray device by performing a generalized calibration routine that is applicable to any rotation center of the x-ray device to measure calibration parameters of the x-ray device that describe at least mechanics of the x-ray device;
imaging a volume by projecting an x-ray beam through the volume onto a detector based on a desired scanning trajectory or the calibration parameters to produce a plurality of x-ray images;
generating an estimated projection geometry corresponding to the imaging using the calibration parameters and the desired scanning trajectory; and
generating a reconstruction of the volume corresponding to the plurality of x-ray images using the estimated projection geometry and a motion artefact compensation.

2. The method of claim 1, wherein a trajectory of the generalized calibration routine is different from the trajectory of any predefined rotation centers.

3. The method of claim 1 or 2, wherein:
the calibration parameters include extrinsic calibration parameters that describe how components of the x-ray device move in space; and
the calibration parameters further include intrinsic calibration parameters that describe relative positions of the components of the x-ray device to each other.

4. The method of claim 3, wherein a trajectory of the generalized calibration routine includes a non-circular segment configured to measure the extrinsic calibration parameters.

5. The method of claim 3 or 4, wherein the generalized calibration routine includes imaging a calibration body with known geometric properties to measure the intrinsic calibration parameters;
optionally wherein: the generalized calibration routine comprises no movement of the x-ray device, and the intrinsic calibration parameters are used in generating the estimated projection geometry.

6. The method of any one of claims 1 to 5, wherein the reconstruction includes at least one of a CBCT (Cone beam computed tomography) volume, a panoramic x-ray image and a cephalometric image.

7. The method of any one of claims 1 to 6, wherein the motion artefact compensation corrects artefacts in the reconstruction caused by errors in estimating the estimated projection geometry.

8. The method of any one of claims 1 to 7, further comprising:
using position encoders or light barriers to obtain additional position information about components of the x-ray device, and
generating the estimated projection geometry based on the additional position information.

9. The method of any one of claims 1 to 8, wherein the desired scanning trajectory takes into account an image quality consideration for achieving a predetermined image quality;
optionally wherein the image quality consideration is a speed consideration or a sensor timing consideration.

10. An x-ray device comprising:
a processor; and
a memory, in communication with the processor, with one or more computer program instructions stored on the memory, the computer program instructions, when executed by the processor, cause the x-ray device to perform operations comprising:
calibrating an x-ray device by performing a generalized calibration routine that is applicable to any rotation center of the x-ray device to measure calibration parameters of the x-ray device that describe at least mechanics of the x-ray device;
imaging a volume by projecting an x-ray beam through the volume onto a detector based on a desired scanning trajectory or the calibration parameters to produce a plurality of x-ray images;
generating an estimated projection geometry corresponding to the imaging using the calibration parameters and the desired scanning trajectory; and
generating a reconstruction of the volume corresponding to the plurality of x-ray images using the estimated projection geometry and a motion artefact compensation.

11. The x-ray device of claim 10, wherein:
the calibration parameters include extrinsic calibration parameters that describe how components of the x-ray device move in space; and
the calibration parameters further include intrinsic calibration parameters that describe relative positions of the components of the x-ray device to each other.

12. The x-ray device of claim 11, wherein the computer program instructions, when executed by the processor, cause the x-ray device to perform operations comprising: configuring a trajectory of the generalized calibration routine to include a non-circular segment configured to measure the extrinsic calibration parameters; and/or
when executed by the processor, cause the x-ray device to perform operations comprising: configuring the generalized calibration routine to image a calibration body with known geometric properties to measure the intrinsic calibration parameters.

13. The x-ray device of any one of claims 10 to 12; wherein the computer program instructions, when executed by the processor, cause the x-ray device to perform operations comprising:
correcting, by the motion artefact compensation, artefacts in the reconstruction caused by errors in estimating the estimated projection geometry.

14. The x-ray device of any one of claims 10 to 13; further comprising:
position encoders or light barriers configured to obtain additional position information about components of the x-ray device, and
wherein the computer program instructions, when executed by the processor, cause the x-ray device to perform operations comprising generating the estimated projection geometry based on the additional position information.

15. A non-transitory computer readable storage medium storing computer-readable instruction that when executed by a processor of an x-ray device cause the x-ray device to carry out the method of any one of claims 1 to 9.
